# EUROPEAN PATENT APPLICATION

(11) **EP 1 658 844 A1**
(43) Date of publication of application: **24.05.2006**
(21) Application number: 04105139.2
(22) Date of filing: 19.10.2004
(51) Int. Cl.: A61K 31/35, A61K 31/365

(54) **Use of artemisinin derivatives in the manufacture of a medicament for the treatment of melanoma**

(71) Applicant: Schuler, Gerold, 91080 Spardorf (DE)
(72) Inventor: Schuler, Gerold, 91080 Spardorf (DE)
(74) Representative: Helbing, Jörg

(57) **Abstract**

The present invention provides the use of artemisinin derivatives for preparing a medicament for the treatment of melanoma. The medicament further contains iron providing agents or may be suitable to be administered with such agents.

## Description

The present invention provides the use of artemisinin derivatives for preparing a medicament for the treatment of melanoma, especially metastatic uveal and cutaneous melanoma. The medicament further contains iron providing agents or may be suitable to be administered with such agents.

### Background

Malignant melanomas in humans constitute 1-3% of all malignant tumors, but the global incidence is rising dramatically at a rate of 6-7% per year. The striking upward trend has been evident for several decades. The incidence of melanoma continues to increase faster then any other cancer (Greenlee, R.T. et al., CA Cancer J. Clin. 51(1):15-36 (2001)).
Cutaneous malignant melanoma is an aggressive tumor. Only very thin primary melanomas are cured by excision, while thicker ones even in the absence of clinically detectable local or distant metastases are no longer cured for sure upon excision of the primary tumor (10-year survival rates for melanomas with thickness of ≤ 0.75 mm = 97%, 0.76-1.5 mm = 90%, 1.6-4 mm = 67%, > 4mm = 43%). Upon the occurrence of clinically detectable loco-regional lymph node metastases the 10-year survival rate drops dramatically to 19%, and in case of distant metastases only about 2-3% of patients survive for 10 years (Balch, C. M. et al., Journal of Clinical Oncology 19:3622-3634 (2001); Balch, C. M. et al., Journal of Clinical Oncology 19:3635-3648 (2001)). There is no evidence that any treatment extends survival in patients suffering from distant metastases. Thus, cutaneous malignant melanoma represents a major clinical problem, and new therapeutic approaches are clearly needed, in particular for patients suffering from metastatic disease.

Cutaneous melanoma patients with distant metastases, i.e. Stage IV have a very poor prognosis, as there is no effective therapy available to date that has been proven to extend survival. The expected median survival is only 8.5 months and the chance of long-term survival is only 2-3 % despite therapy (Eton, O. et al., Journal of Clinical Oncology 16:1103-1111 (1998)). Dacarbazine (DTIC) is the standard chemotherapeutic agent approved for the treatment of advanced melanoma ( Chapman, P.B. et al., J. Clin. Oncol. 17:2745-2751 (1999)). It is a nonclassical alkylating agent that undergoes activation in the liver via oxidative metabolism to its active metabolite methyl-triazenon imidazole carboxamide. Dacarbazine produces overall response rates of about 15%. Responses have been observed mainly in soft tissues (skin, lymph nodes, lung), and are usually incomplete and in addition short-lived, ranging from 3 to 6 months. Complete responses are very rare, and even if a complete response occurs at best 25% of these patients experience long-term remissions (Ahmann, D. L. et al., Cancer 63:224-227 (1989)).

While the skin is the most common site for melanoma development, this neoplasm can occur in any tissue that contains melanocytes. The uvea is the second most common site for melanoma development, and uveal melanoma constitute approximately 13% of all malignant melanoma (Egan, K.M. et al., Surv. Ophthalmol. 32(4):239-51 (1988)). With an incidence of 0.79/100000, uveal melanoma is the most common malignant intraocular tumor of adults (Egan, K.M. et al., Surv. Ophthalmol. 32(4):239-51 (1988)). At least 30% of patients with uveal melanoma experience distant metastasis within 10 years usually to the liver, but also to other organs. With distant metastasis, i.e. Stage IV according to American joint committee of cancer (AJCC) (Balch, C.M. et al., J. Clin. Oncol. 19(16):3635-48 (2001)), the prognosis drops dramatically. Only 13% survive more then 1 year and the mean survival time after occurrence of distant metastasis is only 2-5 months (Gragoudas, E.S. et al., Ophthalmology 98(3):383-9 (1991); Mooy, C.M., de Jong P.T., Surv. Ophthalmol. 41(3):215-28 (1996)). Metastatic uveal melanomas usually demonstrate an even worse response to therapy compared to cutaneous melanomas. While Dacarbazine (DTIC), the standard chemotherapeutic agent approved for the treatment of advanced cutaneous melanoma, yields overall response rates of about 15% (Chapman, P.B. et al., J.Clin.Oncol. 17(9):2745-51 (1999)), in uveal melanoma it is far less active with response rates of under 1% (Pyrhonen, S., Eur. J. Cancer 34 Suppl 3:S27-S30 (1998); Meshnick, S.R. et al., Antimicrob. Agents Chemother. 37(5): 1108-14 (1993)). A four drug chemoimmunotherapy regime has been reported to induce considerable objective response and may have contributed to prolonged survival. However, the anti tumor activity of this regime was associated with severe toxicity and therefore a large multicenter trial was terminated (Nathan, F.E. et al., J. Exp. Clin. Cancer Res. 16(2):201-8 (1997)).

Fotemustine is yet another chemotherapeutic agent for the treatment of uveal melanoma. It is a 2-chloroethyl nitrosurea in which an amino acid-phosphoryl adduct has been introduced, resulting in a highly lipophilic molecule with an increased capacity to penetrate into the central nervous system. Based on a case report of 3 uveal melanoma patients (Terheyden, P. et al., Hautarzt 49(10):770-3 (1998)) who experienced a rather favorable outcome and a prolonged survival after treatment with Fotemustine in combination with interferon-a and interleukin-2, a larger trial has been subsequently initiated demonstrating a prolonged survival of treated patients compared to historical controls (Becker, J.C. et al., British Journal of Cancer 87(8):840-5 (2002)).

Metastatic uveal melanoma is extremely difficult to treat and despite reports about some promising chemo-(immuno-)therapy-regimens (Terheyden, P. et al., Hautarzt 49(10):770-3 (1998)), complete responses are the exception and mostly anecdotal (Pyrhonen, S., Eur. J. Cancer 34 Suppl 3:S27-S30 (1998); Woll, E. et al., Melanoma Res. 9(6):575-81 (1999)). The largest prospective trial to date reported only one CR in 48 patients treated, although some beneficial effect of Fotemustine regarding overall survival compared to historical controls was discussed (Becker, J.C. et al., British Journal of Cancer 87(8):840-845 (2002)). However, there is to date no convincing evidence that any therapy leads to an increased overall survival of patients with metastatic uvea (nota bene: or even metastatic cutaneous) melanoma.

In conclusion, there is to date no randomized prospective study available that has proven the benefit of any therapy for metastatic uveal melanoma compared to best supportive care regarding the overall survival. Thus, novel treatment approaches are clearly needed.

Artesunate (A; ART; CAS 88495-63-0; Arsumax, Artesunic acid, Dihydroqinghasu hemsuccinate, Plasmotrin, Qinghaozhi; (3R,5aS,6R,8aS,9R,10S,12R,12aR)-Decahydro-3,6,9-trimethyl-3,12-epoxy-12H-pyrano[4,3-j]1,2-benzodioxepin-10-ol,hydrogen succinate) is a semi synthetic derivative of Artemisinin (B), a compound extracted from Artemisia annua L., also known as "sweet wormwood" or "qinhao" (Chinese for "from green herb"). Artemisinin was isolated by Chinese researchers in 1972 and its structure elucidated in 1979 (Klayman, D.L., Science 228(4703): 1049-55 (1985)).

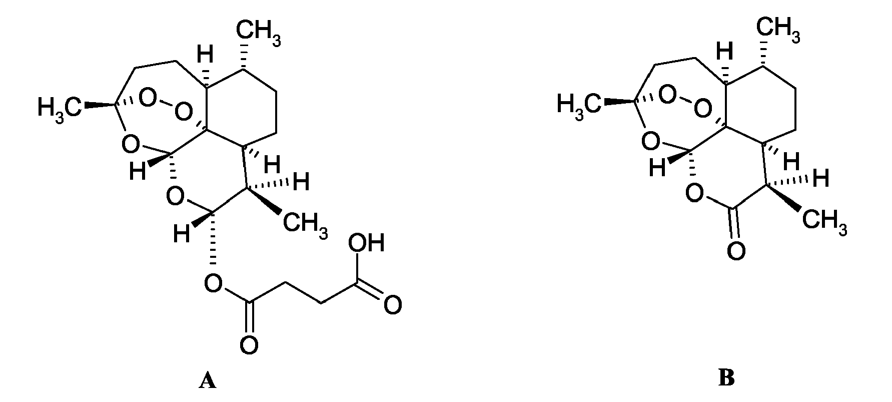

The plant Artemisia annua L., a perennial herb of the family of composite flowers, has been used in traditional Chinese medicine as a remedy for chills and fevers for more then 2000 years. Originally from northern China the plant now grows wild in many countries, though content of artemisinin can vary considerably, depending on plant material and growing conditions (Van Geldre, E. et al., Plant Mol. Biol. 33(2): 199-209 (1997)).

The world health organization recommends the use of artemisinin and its derivatives in geographical areas with multidrug-resistant malaria (van Agtmael, M.A. et al., Trends Pharmacol. Sci. 20(5):199-205 (1999)). Artemisinin acts as blood schizontocide and is able to kill malaria parasites of the genus *Plasmodium.* The endoperoxides to which Artemisinin belongs are a class of antimalarial drugs which meet the dual challenges posed by parasites resistant to other anti-malarial drugs, e.g. chloroquinine, and the rapid progression of malarial illness.

Artemisinin is known as a humoral immunosuppressive agent which is less active than cyclophosphamide, the latter being one of the major chemotherapeutic agents for carcinomas. Artemisinin stimulates cell-mediated immunity, and yet decreases abnormally elevated levels of polyamine regulatory proteins. It also markedly inhibits nucleic acid and protein synthesis. Further it affects cellular membrane functions and decreases hepatic cytochrome oxidase enzyme systemic activity (Tawfik, A.F. et al., Int. J. Immunopharmacol. 12:385-389 (1990); Yang, S.X. et al., Clin. Immunopathol. 69:143-148 (1993); Svensson, U.S. & Ashton, M., Br. J. Clin. Pharmacol. 48:528-535 (1999)).

Artemisinin was also considered virustatic against influenza (US 5,578,637) and cidal against three groups of pathogenic parasites (Efferth, T.H. et al., J. Mol. Med. 80:233-242 (2002)). However, at least the effect against influenza is doubtful (Efferth, T.H. et al., J. Mol. Med. 80:233-242 (2002)).

After structure elucidation of Artemisinin it became obvious that structure adaptation would lead to more stable molecules and to derivatives more suitable for pharmaceutical and therapeutic applications.

Known analogs of artemisinin which have higher solubility in water are dihydroartemisinin, artemether, artesunate, arteether, propylcarbonate dihydroartemisinin and artelinic acid. They all have 100 times the activity of artemisinin in their ability to inhibit nucleic acid synthesis. ART stimulates the immune system at low doses and inhibits it at high doses. Artelinic acid is the most water soluble and most stable of the group. The very low toxicity of theses compounds to humans is a major benefit. ART is twice as safe as artemether and only one-fiftieth as toxic as chloroquinine (US 5,578,637).

Propitious features of ART are the activity against otherwise multidrug-resistant Plasmodium strains, its good tolerability and the lack of serious side effects. ART has been used for the treatment of more than 1 million cases of malaria and is considered a save drug with no obvious adverse reactions or noticeable side effects, even when given to children (van Agtmael, M.A. et al., Trends Pharmacol. Sci. 20(5):199-205 (1999)).

Since the isolation of artemisinin there has been a concerted effort by investigators to study other therapeutic applications of Artemisinin and its derivatives. NIH reported that artemisinin is inactive against P388 leukemia (NCI report on NSC 369397, tested on 25 October 1983). Later anticancer studies that have been conducted on cell line panels consisting of 60 lines organized into nine disease-related subpanels including leukemia, non-small-cell lung cancer, colon, CNS, melanoma, ovarian, renal, prostate and breast cancers, further confirmed that artemisinin displays very little anticancer activity. A series of artemisinin-related endoperoxides were tested for cytotoxicity to Ehrlich ascites tumor (EAT) cells using the microculture tetrazolium (MTT) assay (Woerdenbag, H.J. et al., J. Nat. Prod. 56(6):849-856 (1993)). The MTT assay is based on the metabolic reduction of soluble tetrazolium salts into insoluble colored formazan products by mitochondrial dehydrogenase activity of the tumor cells. Artemisinin, sodium artesunate and other artemisinin derivatives exhibited IC50 values ranging from 12.2 µM to 29.8 µM in this test, i.e. only a very low cytotoxicity.

EP 0882037 describes artemisinin dimer compounds which show antitumor activities which are equivalent to those of paclitaxel. The substances were tested by NCI on their cytostatic and cytotoxic effect on leukemia, non-small cell lung, colon, CNS, melanoma, ovarian, renal, prostate and breast cancer cell lines. The potency of the substances varies from cell line to cell line, but it was at least equivalent to paclitaxel. WO 99/33461 describes trioxane Artemisinin analogs and WO 00/42046 describes olefinic analogs thereto, some of which show antiproliferative and antitumor activities which are equivalent to those of known antiproliferative and antitumor agents. These substances are monomers and dimers of a trioxane structure similar to artemisinin. Their antiproliferative effect to murine keratinocyte cell line PE was tested and resulted in substances which were at least as antiproliferative as calcitrol which is used clinically as a drug to treat psoriasis, a skin disorder characterized by uncontrolled proliferation of cells. These substances were then tested by NCI on their cytostatic and cytotoxic effect on leukemia, non-small cell lung, small cell lung, colon, CNS, melanoma, ovarian, renal, prostate and breast cancer cell lines. Comparison substances were artemisinin and paclitaxel. The potency of the substances tested in WO 00/42046 and WO 99/33461 varies from cell line to cell line, but they were more potent than artemisinin and at least equivalent to paclitaxel. However, in none of WO 00/42046, WO 99/33461 and EP 0882037 a comparison to ART was made and no uveal melanoma cells were tested.

WO 2004/071506 describes the use of Artemisinin-related compounds including Artesunate for treating proliferative cervical disorders such as cervical cancer induced by oncogenic viruses.

EP 0428773 and US 4,978,676 disclose the treatment of psoriasis, viral- or UV-radiation induced skin diseases and skin tumors and malignant melanoma with artemisinin and some artemisinin derivatives including artesunate. US 5,219,880 discloses the treatment of hemorrhoids with artemisinin and its derivatives, including artesunate and artesunate salts. However, in each of these patents just one example using artemisinin in treatment of psoriasis/hemorrhoids is given and the use of all claimed substances in the other diseases mentioned is merely hypothetical.

It was recently demonstrated that ART, apart from its anti-malarial activity, inhibits the growth of leukemic cells (Efferth, T. et al., Arzneimittelforschung 46(2):196-200 (1996)). Subsequently it was shown that ART is also active against a variety of human cancer cells lines including melanoma cells (Efferth, T. et al., Int. J. Oncol. 18(4):767-73 (2001)). In addition, the molecular mode of action has been elucidated (Efferth, T. et al., Mol. Pharmacol. 64(2):382-94 (2003); Efferth, T. et al., Int. J. Oncol. 23 :1231-1235 (2003); Efferth, T. et al., Biochem. Pharmacol. 64:617-623 (2002)). Importantly, a comparison of ART's cytotoxicity with those of other standard cytostatic drugs showed that the effect of ART is based on the activation of other oxidative genes as the effect of, e.g., anthracyclins, although ART as well as anthracyclin cause the formation of radicals or reactive oxygen species. Furthermore, besides the side effects typical for cytostatica, anthracyclins possess cardiotoxic properties, whilst artemisinin-derivatives are nearly free of negative side effects (Efferth, T. & Oesch, F., Biochem. Pharmacol. 68:3-10 (2004)).

Moreover, in comparison experiments ART was active in molar ranges comparable to those of established anti-tumor drugs. Of note, the concentration of ART applied in these *in vitro* studies was significantly lower compared to serum levels usually accomplished in anti-malaria therapy. Other independent studies have confirmed these findings and additionally demonstrated antiangiogenic effects of ART (Reungpatthanaphong, P. & Mankhetkorn, S., Biol. Pharm. Bull. 25(12):1555-61 (2002); Chen, H.H. et al., Pharmacol. Res. 48(3):231-6 (2003)). Besides numerous reports describing the in vitro activity of ART against cancer cells (Efferth, T. et al., Arzneimittelforschung 46(2):196-200 (1996); Efferth, T. et al., Int. J. Oncol. 18(4):767-73 (2001); Lai, H. & Singh, N.P., Cancer Lett. 91(1):41-6 (1995); Singh, N.P. & Lai, H., Life Sci. 70(1):49-56 (2001)), there is only a single case report to date in which ART was applied to treat human cancer (Singh, N.P. &Verma, K.B., Archive of Oncology (2002)).

Higher efficacy of artemisinin action can be achieved by utilization of the fact that it is more reactive with heme than free iron (Hong et al., Mol. Biochem. Parasit. 63:121-128 (1974)). Heme can be introduced into cells using transferrin (Stout, D.L., Biochim. Biophys. Res. Comm. 189:765-770 (1992)) or a heme-carrying compound. In the presence of heme, the endoperoxide bridge of artemisinin and its analogs undergoes reductive decomposition to form a free radical and electrophilic intermediates. US 5,578,637 describes the use of this principle to enhance the inhibition or killing of cancer cells with artemisinin and its analogs or endoperoxide compounds other than artemisinin (like arteflene and its derivatives and 1,2,4-trioxanes as well as 1,2,4,5-tetraoxanes) as therapeutic agents. According to US 5,578,637, iron agents useful for enhancing intracellular iron concentrations include pharmaceutically acceptable iron salts and iron complexes. In said patent, Dihydroartemisinin and holotransferrin were applied to molt-4 human lymphoblastoid cells and normal human lymphocytes as comparison cells. Dose-dependent decrease of cell counts was observed, which showed that combined incubation in holotransferrin and dihydroartemisinin can selectively destroy the molt-4 lymphoblastoid cells. Moreover three *in vivo* dog studies showed that an combination treatment with ferrous sulfate and artemisinin leads to reduction of tumor size and prevents tumor recurrence. Cancer treatment using artemisinin or its derivatives in combination with iron providing or iron enhancing agents was therefore introduced by said US 5,578,647, including use of the procedure in prophylactic cancer prevention, prevention of cancer recurrence and metastases after surgery, and as an adjuvant of other traditional cancer therapy.

The mode of action of ART becomes apparent in the presence of free iron. It parallels the mode of action of artemisinin in cancer cells. Fe²⁺ catalyses the opening of the endoperoxide bridge, leading to the formation of very reactive free radicals which cause extensive damage of either parasites or cancer cells (Meshnick, S.R. et al., Antimicrob. Agents Chemother. 37(5):1108-14 (1993); Kamchonwongpaisan, S. & Meshnick, S.R., Gen. Pharmacol. 27(4):587-92 (1996)). Since tumor cells sequester relatively large amounts of iron compared to normal cells, they are more susceptible to the cytotoxic effects of ART. It is also conceivable to further increase the activity of ART by loading cancer cells with iron, e.g. by co-administration of iron supplementing medication.

### Summary of the Invention

Surprisingly it was now found that artemisinin derivatives are suitable for the treatment of several melanoma, especially in combination with iron enhancing agents. Thus, the invention provides
(1) the use of an artemisinin derivative of the formula (I) hereinafter "compound (I)" 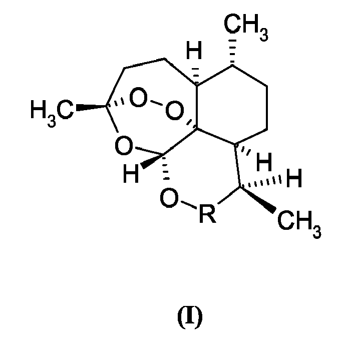 wherein R is 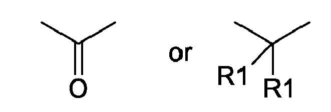 wherein each of R1 are independently selected from hydrogen, hydroxyl, alkyl, alkoxyl, alkylaryl, aryl or the formula: 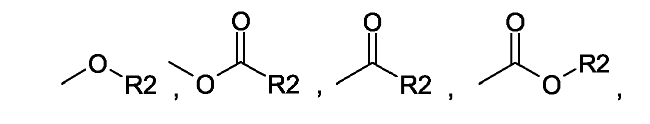 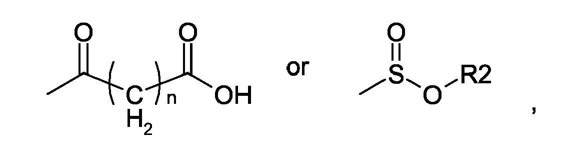 wherein R2 is alkyl or aryl and n is 1 to 6,
   or a pharmaceutically acceptable salt thereof for preparing a medicament for the treatment of metastatic uveal melanoma;
(2) the use of compound I or a pharmaceutically acceptable salt thereof in combination with an iron enhancing agent effective to increase the intracellular iron concentration of the patient for preparing a medicament for the treatment of cutaneous melanoma;
(3) in a preferred embodiment of (1) and (2) the artemisinin derivative is artesunate;
(4) in a further preferred embodiment of (1) the medicament further comprises an iron enhancing agent effective to increase the intracellular iron concentration of the patient, or is suitable to be administered in combination with such iron enhancing agent;
(5) a method for the treatment of metastatic uveal or cutaneous melanoma in a patient, which comprises administering to the patient a suitable amount of an artemisinin derivative as defined in (1) or (3) above.

### Brief description of the figures

The following figures are provided in order to explain further the present invention in supplement to the detailed description and the examples:
Fig. 1 illustrates the clinical course of the disease and the treatment modalities in the *in vivo* study described in detail in Example 1.
Fig. 2 illustrates the clinical course of the disease and the treatment modalities in the *in vivo* study described in detail in Example 2.

### Detailed description of the invention

In the framework of the present invention the following terms and definitions are used.

The term "melanoma" describes the disease in which malignant cells are formed from melanocytes.

The term "uveal melanoma" means the most common primary intraocular malignancy in adults, which starts with development of melanoma in the uveal region. It is almost invariably unilateral and has its highest incidence between the fifth and seventh decades of age. It arises most frequently in the choroid, less frequently in the ciliary body and in the iris. The tumor initially grows flat within the choroid, later it elevates Bruch's membrane and finally it ruptures through it so that the melanoma assumes a characteristic mushroom-shape growing toward the vitreous humor. The retina over the surface of the tumor becomes elevated and detached (solid retinal detachment). This detachment gradually extends as a serous detachment over the slopes of the tumor. Uveal melanoma may precociously metastasize at distant sites including liver, lung, bone, kidney and brain in order of decreasing frequency. Extraocular orbital extension by contiguous spread also occurs if the lesion is left untreated.

The term "cutaneous melanoma" defines the disease in which melanoma start in the skin. In detail, cutaneous melanoma is a neoplasm arising from the melanocytes that can occur on the skin *de novo,* or from a pre-existing lesion such as a congenital, acquired, or atypical (dysplastic) nevus. Non-cutaneous primary sites of melanocytes also include the mucosal epithelium, retina, and leptomeninges.

The terms "alkyl" and "alkoxyl" refer to linear, branched or cyclic alkyl residues which may optionally contain one or more double or triple bond. Alkyl or alkoxyl residues comprising from 1 up to 6 carbon atoms, are preferred, especially preferred are those residues comprising from 1 up to 3 carbon atoms.

The term "aryl" refers to monocyclic or bicyclic aromatic ring systems comprising 4-7 ring atoms, preferably 5- and 6-membered rings. Especially preferred is phenyl.

The present invention provides the use of artemisinin derivatives of the formula (I) as defined hereinbefore (compound (I)) for preparing a medicament for treating metastatic uveal melanoma. Preferred compounds (I) are selected from dihydroartemisinin, artemether, artesunate, arteether, propylcarbonate dihydroartemisinin and artelinic acid, the most preferable compound is artesunate.

Iron agents useful for enhancing intracellular iron concentration according to embodiment (2) and (4) include pharmaceutically acceptable iron salts and iron complexes, including ferrous chloride, ferrous fumarate, ferrous sulfate, ferrous carbonate, ferrous citrate, ferrous gluconate, ferrous glutamate, ferrous succinate, ferrous lactate, ferrochinolate, ferroglycine sulfate, dextran iron complex, peptonized iron, iron sorbitex, ferric oxide, saccharated iron, holoferritins and holotransferrins, preferably ferroglycine sulfate, ferrous gluconate and ferrous sulfate. Especially preferred is ferroglycine sulfate.

The combination of an artemisinin derivative of present invention, most preferably ART, to one or more classical chemotherapeutic agents as a means of further enhancing clinical efficacy is another embodiment of the medicament according to (1) and (2) and the method according to (5). Thus, the formulations for present invention may further include as optional ingredients one or more agents already known for their use in inhibition of cancer cells. Alternatively, the medicament according to (1) and (2) may be administered in combination with one or more of these known cancer inhibiting agents. Known cancer inhibiting agents are androgen inhibitors, antiestrogens, cytotoxic agents, hormones, nitrogen mustard derivatives, steroids and the like. This may comprise administering to the patient an antineoplastic agent selected from e.g. Fotemustine, Dacarbazin, Cisplatinum, Carmustine, Trofosfamide, Docetaxel, Bleomycin, Vincristin, especially Fotemustine and/or Dacarbazin at any stage of the therapy in parallel and/or alternating with the medicament according to (1) or (2).

The medicament according to (1) and (2) and the method according to (5) are suitable for long term treatment of metastatic cutaneous and uveal melanoma patients, optionally in combination with chemotherapy and/or with conventionally used antineoplastic agents as listed above.

The method of embodiment (5) may comprise the administering of an iron enhancing agent to the patient prior to, at the same time or after administration of the artemisinin derivative. The iron enhancing agent may be administered once or several times daily, preferably 2-4 times daily, most preferably three times daily.

The concentrations of agents for enhancing intracellular iron concentrations will generally range up to the maximally tolerated dose for a particular subject and agent, which will vary depending on the agent, subject, disease condition and so forth. Dosages ranging from 40 to 300 mg daily dose Fe²⁺ will generally be useful for this purpose, preferably from 80-240 mg daily dose Fe²⁺, most preferably 120 mg of iron per day given in three doses à 40 mg Fe²⁺.

The concentrations of the artemisinin derivatives in the formulations of the medicament according to (1) and (2) will generally range up to the maximally tolerated dosage. Best results will be obtained using formulations containing the compounds at levels of 1-4 mg per kg body weight per day, preferably 1-3 mg per kg body weight per day, most preferably 1.5-2 mg per kg body weight per day. The precise amounts employed will vary depending on the compound, the route of administration, the physical condition of the patient and other factors. The daily dosage may be administered as a single dosage or may be divided into multiple doses for administration, whereby a multiple dosage of 2-3 times per day is preferred.

The medicament according to (1) and (2) may further comprise a pharmaceutically acceptable carrier, excipient and/or auxiliary agent. Any conventional formulation may be used for this medicament, provided that the carrier does not significantly interfere with the stability or bioavailability of compound (I) and/or the iron enhancing agent.

The medicament according to (1) and (2) and the iron enhancing agent according to (4) may be administered topically or systemically by oral, lavage, suppository, parenteral, intramuscular or intravenous administration, especially orally.

The present invention is hereinafter described with reference to Artesunate (ART). It was found that the therapy of two patients suffering from metastatic uveal melanoma with ART in combination with chemotherapy was well tolerated with no additional side effects other than that caused by the chemotherapy alone. One patient experienced a temporary response after addition of ART to Fotemustine while under therapy with Fotemustine alone the disease was progressing. The second patient first experienced a stabilization of the disease after addition of ART to Dacarbazin followed by objective regressions of all but one residual lung metastases. This patient is still alive 37 months after first diagnosis of stage IV uveal melanoma, a situation with a median survival of 2-5 months. Thus, ART might be, in combination with chemotherapy, a promising adjuvant drug for the treatment of melanoma. Its well tolerability and lack of serious side effects will facilitate prospective randomized trials in the near future. Firstly, ART-treatment was without measurable toxicity. Secondly, one of the treated patients exhibited a favorable clinical course of disease with an actual survival of 37 months, which is in consideration of the extremely poor prognosis of this diagnosis most remarkable.

Both patients were suffering from metastatic uveal melanoma and were progressing despite a standard chemotherapy with DTIC or Fotemustine. It was not possible to include these patients in actual therapeutic trials, since respective protocols were not available. Therefore applicant proposed, based on the *in vitro* evidence of cytotoxic activity of ART against melanoma cells (Efferth, T. et al., Int. J. Oncol. 18(4):767-73 (2001)) and the excellent safety record of ART, a "compassionate-use treatment" with ART in combination with ongoing chemotherapy. The therapy was well tolerated in both patients with no detectable side effects except that caused by the concomitant chemotherapy. In patient 1, the addition of ART to Fotemustine was followed by temporary mixed response to therapy with regression of some lung metastases, but development of new CNS-tumors. The patient survived 11 months after start of the ART/Fotemustine therapy, which is significantly longer than the median survival time of these patients. The second patient had only a moderate progress of his metastatic disease before addition of ART to DTIC, however, he had a stable disease after start of ART/DTIC and subsequently achieved a objective response with regression of the splenic metastasis and all but one lung metastasis. This patient is still alive without any symptoms of his disease, 37 months after initiation of ART/DTIC-therapy.

In the following examples, material and methods of the present invention are provided including the treatment of human patients using said material and methods. It should be understood that these examples are for illustrative purpose only and are not to be construed as limiting this invention in any manner. All publications, patents, and patent applications cited herein are hereby incorporated by reference in their entity for all purposes.

### Examples

### Materials and methods

In the experimental examples below, standard techniques of therapeutical agent administration were used.
The patients were treated on a compassionate-use basis, after standard chemotherapy alone was not effective to stop tumor growth.
Artesunate was provided by Sanofi Synthelabo, France.

### Example 1: In vivo human study 1

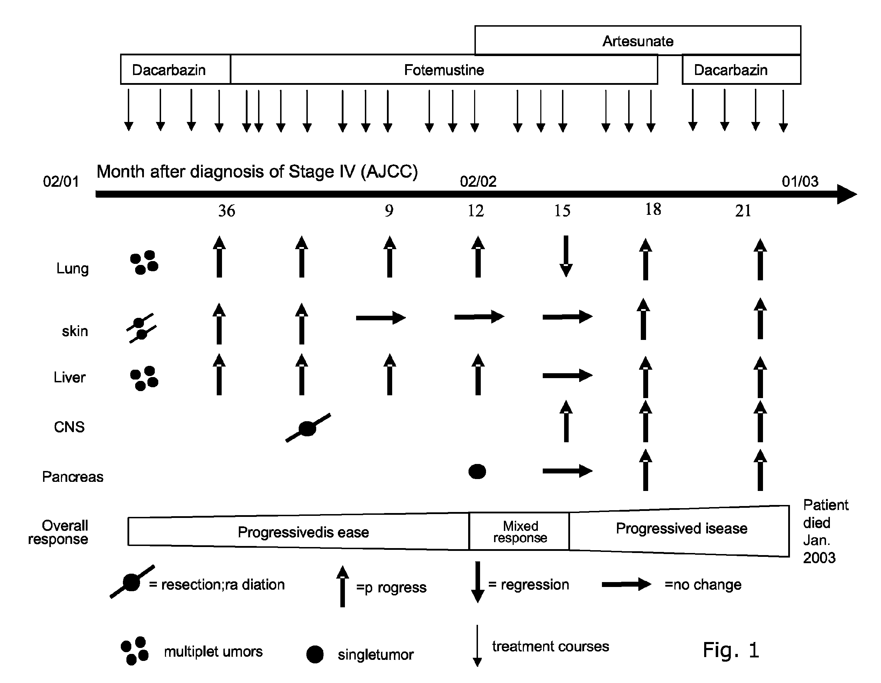

The clinical course of the disease and the treatment modalities are depicted in detail in Figure 1.
Patient 1, a female born 1932, was diagnosed with uveal melanoma in August 1996. Her left eye was enucleated, subsequently she was in regular control without pathologic findings until February 2001, when distant metastases were first diagnosed. On examination, she was in good general health. 2 small skin tumors were found on the scalp and the left axillary region respectively, a CT-scan revealed multiple lung metastases on both sides as well a multiple liver metastases. A chemotherapy with Dacarbazin (DTIC) was initiated at a concentration of 850 mg per m² body surface. After 3 months and 4 treatment courses (1 treatment course = 1 day; treatment courses were generally separated by 4±0.5 weeks), a CT-staging showed a progressive disease, therefore DTIC was stopped and a chemotherapy with Fotemustine at a concentration of 100 mg per m² body surface was started. After three months and 4 treatment courses (1 treatment course = 1 day; treatment courses were generally separated by 4±0.5 weeks, the very first one took place one week before the second course), the disease was moderately progressing, although a CNS-metastasis was diagnosed and had to be treated by stereotactic irradiation as well as 3 new skin tumors on the scalp were detected and surgically removed. After 3 additional treatment courses, the known metastases were slightly increasing in size, but since no new organs became involved, we proceeded with the Fotemustine therapy. However, after another 3 treatment courses, in February 2002 a further progress was diagnosed with known metastases increasing in size and number as well as involvement of another organ, the pancreas. Due to a lack of a standard regimen after 2 chemotherapeutics had failed and after receiving informed consent of the patient we started a compassionate treatment with additional daily ART (2 times daily 50 mg p.o.) in combination with Fotemustine. The first staging after three treatment courses of Fotemustine in combination with ART three months after the initiation of this therapy-mode showed a mixed response. Whereas in the liver there were no significant changes, the lung metastases were decreasing in number and there was no evidence for new metastases neither in visceral organs nor at the skin. However, two new CNS-tumors were diagnosed by MRT and subsequently irradiated. After this phase of relative stabilization of disease progression, the following staging demonstrated a significant progress. We therefore changed the chemotherapy to DTIC, but continued with ART. However, the disease was further progressing and the patient died in January 2003, 23 months after entry into stage IV according to AJCC (Balch, C.M. et al., J. Clin. Oncol. 19(16):3635-48 (2001)). Throughout the combination therapy, there were no additional side effects than that caused obviously by the chemotherapy alone as nausea and vomiting and bone marrow insufficiency leading to anemia, leucopenia and thrombopenia with the expected latency after the chemotherapy treatment courses.

### Example 2: In vivo human study 2

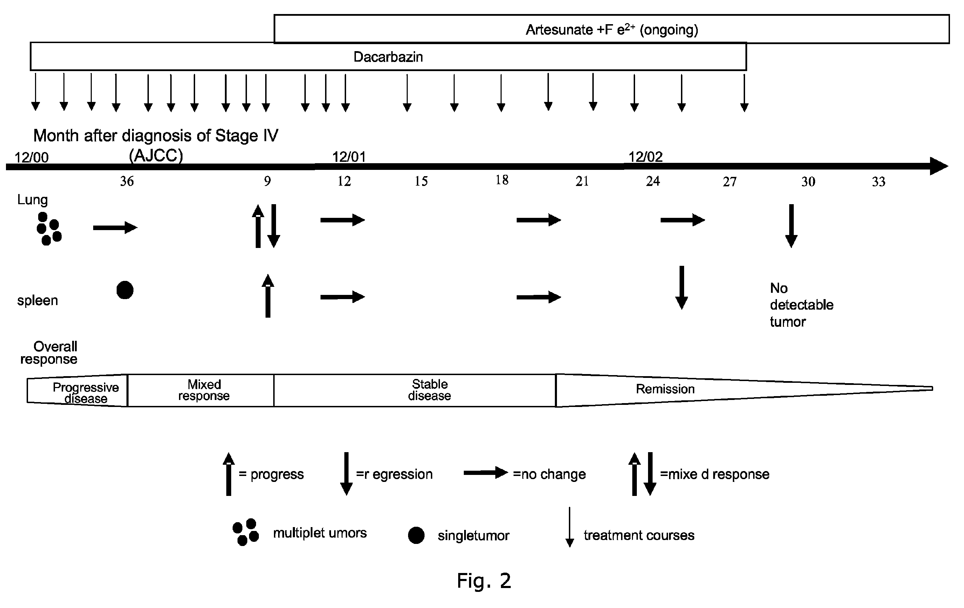

The clinical course of the disease and the treatment modalities are depicted in detail in Figure 2.
Patient 2, a 1926 born male had a left eye uveal melanoma diagnosed in November 1987, which was treated by enucleation and subsequent radiotherapy. Without a regular follow-up the patient was healthy until November 2000, when by chance round structures were seen in a routine chest X-ray. The histopathological examination of a tumor biopsy revealed melanoma metastases. At that time, there were no other metastases detectable. A chemotherapy with DTIC at a concentration of 850 mg per m² body surface was initiated. After a phase of relatively stable or only slowly progressing disease for 9 months (Fig. 2), a marked enlargement of a previously hardly detectable spleen metastasis was observed. Because the lung metastases showed a mixed response and no other organs became involved, we decided to continue with DTIC (1 treatment course = 1 day; treatment courses were generally separated by 4±0.5 weeks), but started a concomitant therapy with 100 mg ART daily (50 mg twice daily p.o.), after the patient gave his informed consent. In addition, the patient received an iron medication (three times daily ferroglycine sulfate equivalent to 40 mg Fe²⁺) to potentially increase the efficacy of ART. In the following months the disease first became stable, followed by an objective response first seen after 21 treatment courses of DTIC (the last 11 of which in combination with ART), when all but one lung and the spleen metastases completely disappeared. The patient has received a last treatment course of DTIC in April 2003 and is free of symptoms under sole medication with ART since.

## Claims

1. Use of an artemisinin derivative of the formula (I) 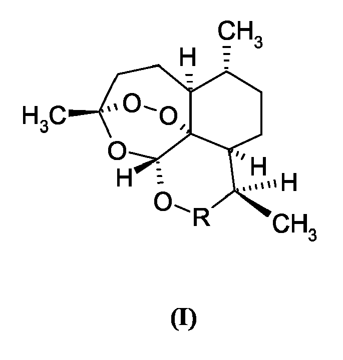 wherein R is 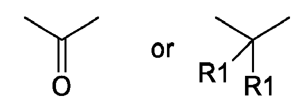 wherein each of R1 are independently selected from hydrogen, hydroxyl, alkyl, alkoxyl, alkylaryl, aryl or the formula: 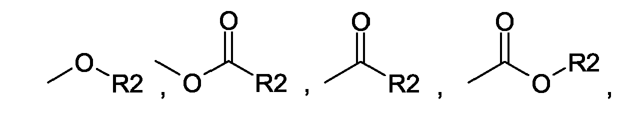 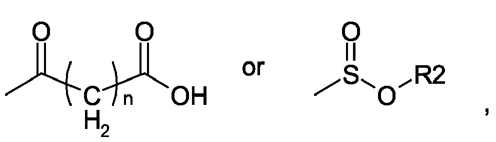 wherein R2 is alkyl or aryl and n is 1 to 6,
or a pharmaceutically acceptable salt thereof for preparing a medicament for the treatment of metastatic uveal melanoma.

2. The use of claim 1, wherein the medicament further comprises an iron enhancing agent effective to increase the intracellular iron concentration of the patient, or is suitable to be administered in combination with such iron enhancing agent.

3. Use of an artemisinin derivative of the formula (I) as defined in claim 1 or a pharmaceutically acceptable salt thereof for preparing a medicament for the treatment of cutaneous melanoma, wherein the medicament further comprises an iron enhancing agent effective to increase the intracellular iron concentration of the patient, or is suitable to be administered in combination with such iron enhancing agent.

4. The use of claim 2 or 4 wherein the iron enhancing agent is selected from pharmaceutically acceptable iron salts and iron complexes, preferably from the group consisting of ferrous fumarate, ferrous sulfate, ferrous carbonate, ferrous citrate, ferrous gluconate, ferrous glutamate, ferrous succinate, ferrous lactate, ferrochinolate, ferroglycine sulfate, dextran iron complex, peptonized iron, iron sorbitex, ferric oxide, saccharated iron, holoferritins and holotransferrins, most preferably is ferroglycine sulfate.

5. The use of claim 1 or 2, wherein the artemisinin derivative has a higher solubility in water, preferably is selected from dihydroartemisinin, artemether, artesunate, arteether, propylcarbonate dihydroartemisinin and artelinic acid, most preferably is artesunate.

6. The use according to any of claims 1 to 5 where the medicament is suitable to be administered topically or systemically by oral, intramuscular or intravenous administration.

7. A method for the treatment of metastatic uveal melanoma or cutaneous melanoma in a patient, which comprises administering to the patient a suitable amount of an artemisinin derivative of the formula I as defined in claim 1 or 5.

8. The method according to claim 7, wherein an iron enhancing agent, preferably as defined in claim 4, is administered to the patient (i) prior to, (ii) at the same time or (iii) after administration of the artemisinin derivative.

9. A method for the treatment of cutaneous melanoma in a patient which comprises administering to the patient a suitable amount of an artemisinin derivative of the formula I as defined in claim 1 or 5, and an iron enhancing agent, preferably as defined in claim 4, where the iron enhancing agent is administered (i) prior to, (ii) at the same time or (iii) after administration of the artemisinin derivative.

10. The method of claim 8 or 9 comprising administering to said patient 40 to 240 mg daily dose Fe²⁺, preferably from 60-180 mg daily dose Fe²⁺, most preferably 120 mg daily dose Fe²⁺ or 40 mg Fe²⁺ three times daily.

11. The method of claims 7 to 10 which further comprises administering to the patient an antineoplastic agent, preferably selected from Fotemustine, Dacarbazin, Cisplatinum, Carmustine, Trofosfamide, Docetaxel, Bleomycin, Vincristin, most preferably selected from Dacarbazine and/or Fotemustine.
